# EUROPÄISCHE PATENTANMELDUNG

(11) **EP 0 785 253 A1**
(43) Veröffentlichungstag der Anmeldung: **23.07.1997**
(21) Anmeldenummer: 96120947.5
(22) Anmeldetag: 27.12.1996
(51) Int. Cl.: C12N 15/12, C07K 14/435, C07K 1/14, C12Q 1/68, G01N 33/68, A61K 39/00

(54) **Dictyocaulus viviparus Antigen zur Diagnose des Lungenwurmbefalls und zur Vakzinierung**

(30) Priorität: 19.01.1996 DE 19601754
(71) Anmelder: HOECHST AKTIENGESELLSCHAFT, 65929 Frankfurt am Main (DE)
(72) Erfinder: Hofmann, Joachim, Dr. Ing., 65520 Bad Camberg (DE); Schmid, Karlheinrich, Dr., 65719 Hofheim (DE)

(57) **Zusammenfassung**

Die Erfindung betrifft ein immunogenes Protein (DV18) aus dem Lungenwurm Dictyocaulus viviparus mit protektiver Wirkung, Verfahren zu dessen Isolierung, Verfahren zur Isolierung der zugehörigen DNA-Sequenz, Herstellung des Proteins auf gentechnologischem Wege, Verwendung des Proteins in Diagnostik-Kits und als Vakzine.

SEQUENZPROTOKOLL

(1) ALLGEMEINE INFORMATION:

(i) ANMELDER:
(A) NAME: Hoechst Aktiengesellschaft
(B) STRASSE: -
(C) ORT: Frankfurt
(D) BUNDESLAND: -
(E) LAND: Deutschland
(F) POSTLEITZAHL: 65926
(G) TELEPHON: 069-305-7433
(H) TELEFAX: 069-35-7175
(I) TELEX: 412 34 700 ho d

(ii) ANMELDETITEL: Dictyocaulus viviparus Antigen zur Diagnose
des Lungenwurmbefalls und zur Vakzinierung

(iii) ANZAHL DER SEQUENZEN: 8

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 17 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..17

(xi) SEQUENZBESCHREIBUNG: SEQ ID NO: 1:

Thr Pro Pro Ala Gly Val Phe Tyr Gln Gly Xaa Ser Ala Thr Pro Ile
1               5                   10                  15

Ala

(2) INFORMATION ZU SEQ ID NO: 2:

(i) SEQUENZ CHARAKTERISTIKA:
(A) LÄNGE: 20 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..20

(xi) SEQUENZBESCHREIBUNG: SEQ ID NO: 2:

Xaa Leu Gly Val Asp Pro Ala Ser Gly Val Leu Asp Pro Lys Glu Ala
1               5                   10                  15

Thr Leu Met Ala
            20

(2) INFORMATION ZU SEQ ID NO: 3:

(i) SEQUENZ CHARAKTERISTIKA:
(A) LÄNGE: 8 Aminosäuren
(B) ART: Aminosäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: Protein
(B) LAGE: 1..8

(xi) SEQUENZBESCHREIBUNG: SEQ ID NO: 3:

Asn Leu Pro Ile Glu Tyr Asn Pro
1               5

(2) INFORMATION ZU SEQ ID NO: 4:

(i) SEQUENZ CHARAKTERISTIKA:
(A) LÄNGE: 16 Aminosäuren
(B) ART: Aminosäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: Protein
(B) LAGE: 1..16

(xi) SEQUENZBESCHREIBUNG: SEQ ID NO: 4:

Ala Phe Arg Arg Glu Trp Phe Gln Gly Asp Gly Met Val Arg Arg Lys
1               5                   10                  15

(2) INFORMATION ZU SEQ ID NO: 5:

(i) SEQUENZ CHARAKTERISTIKA:
(A) LÄNGE: 10 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Active-site
(B) LAGE: 1..10

(xi) SEQUENZBESCHREIBUNG: SEQ ID NO: 5:

Asp Val Phe Asn Ala Pro Tyr Asp Asp Lys
1               5                   10

(2) INFORMATION ZU SEQ ID NO: 6:

(i) SEQUENZ CHARAKTERISTIKA:
(A) LÄNGE: 22 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..22

(xi) SEQUENZBESCHREIBUNG: SEQ ID NO: 6:

Xaa Pro Pro Ala Gly Val Phe Tyr Gln Gly Trp Ser Ala Thr Pro Ile
1               5                   10                  15

Ala Asn Gly Ser Leu Gly
            20

(2) INFORMATION ZU SEQ ID NO: 7:

(i) SEQUENZ CHARAKTERISTIKA:
(A) LÄNGE: 5 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..5

(xi) SEQUENZBESCHREIBUNG: SEQ ID NO: 7:

Thr Tyr His Ile Lys
1               5

(2) INFORMATION ZU SEQ ID NO: 8:

(i) SEQUENZ CHARAKTERISTIKA:
(A) LÄNGE: 14 Aminosäuren
(B) ART: Aminosäure
(C) STRANGFORM: Einzel
(D) TOPOLOGIE: linear

(ii) ART DES MOLEKÜLS: Protein

(ix) MERKMALE:
      (A) NAME/SCHLÜSSEL: Protein
      (B) LAGE: 1..14

(xi) SEQUENZBESCHREIBUNG: SEQ ID NO: 8:

Ile Ile Asn Ala Ser Gly Arg Arg Ile Gly Trp Ala Ile Lys
1               5                   10

## Beschreibung

Die Erfindung betrifft ein Antigen aus den adulten Stadien des Lungenwurms Dictyocaulus viviparus (im folgenden auch D. viviparus oder Dictyocaulus genannt) des Rindes, mit dem man immundiagnostisch Lungenwurmbefall bei Rindern nachweisen kann. In einer Vakzine kann das Antigen Immunschutz gegen D. viviparus hervorrufen.

Lungenwürmer haben insbesondere bei kleinen und großen Wiederkäuern eine große pathogene und wirtschaftliche Bedeutung. Dictyocaulus ist der einzige beim Rind Geschlechtsreife erlangende Lungenwurm. Er kommt weltweit dort vor, wo zumindest zeitweise gemäßigte Temperaturen von 15-20°C herrschen. Endemisch ist D. viviparus in Europa in den großen Flußauen, regenreichen Küstengebieten aber auch auf alpinen Almen verbreitet (R.J. Jörgensen (1980) Vet. Parasitol. 7, 153-167; H. Pfeiffer (1976) Wien. Tierärztl. Mschr. 63, 54-55). In den Niederlanden wurde z.B. bei über 77% der auf Weiden gehaltenen Kälbergruppen klinische Dictyokaulose festgestellt (J. Boch, R. Supperer (1992) Veterinärmedizinische Parasitologie. 4. Aufl., Parey, Berlin, S. 294-301.)

Die Aufnahme der dritten Larven mit dem Weidegras verursacht beim erstmals exponierten Kalb die Erkrankung (Dictyokaulose). Die Larven erreichen auf dem Blutweg die Alveolen der Lungen, die sie durchbrechen, um in die luftführenden Teile der Lunge zu gelangen. Dabei werden Läsionen erzeugt, die als Eintrittspforte für bakterielle Sekundärinfektionen dienen; die Vermehrung von Bakterien und anderer mikrobieller Erreger führt zu begrenzten oder generalisierten Lungenentzündungen mit allen möglichen Folgeerscheinungen wie Lungenödem und Herzversagen (T. Schnieder, A. Bellmer, F.-J. Kaup (1989) Wien. Tierärztl. Mschr. 76:372-476). Die Atmung wird, auch durch die in den oberen Atmungswegen zu Obstruktionen führenden adulten Stadien, erheblich erschwert. Sichtbare Folgen der starken Beeinträchtigung des Allgemeinbefindens sind verminderte Gewichtszunahmen oder gar Gewichtsverluste verbunden mit Wachstumsverzögerungen. Bisweilen verschlimmern sich die klinischen Symptome dramatisch und führen rasch zum Tod.

Die Lungenwurmerkrankung der Rinder kann anhand der klinischen Symptomatik (G. Gräfner (1987) Monatsh. Vet. med. 42:178-181) oder anhand der mit dem Kot ausgeschiedenen Larven diagnostiziert werden (J. Boch, R. Supperer (1992)). Diese Möglichkeiten eignen sich vor allem für die Diagnose am Einzeltier, wenn eine starke Infektion vorliegt. Zu der modernen Massentierhaltung werden jedoch, basierend auf einer geeigneten Diagnostik, epidemiologische Voraussagen und Risikoeinschätzungen bezüglich des Ausbruchs einer Dictyokaulose in der fortgeschrittenen Weidesaison benötigt; d.h. in Surveys müssen viele, möglicherweise noch schwach infizierte Kälber mit einer sicheren, sensiblen Methode untersucht werden. Hierzu sind serologische Methoden geeignet (A. Bellmer, T. Schnieder, A.M. Tenter (1989) Proc. 13th Conf. Wrld Ass. Adv. Vet. Parasit., S. 33, Berlin, 7.-11.8.1989). In Dictyocaulus viviparus identifizierte, isoliert und teilweise rekombinant dargestellte Antigene werden zur Serodiagnostik genutzt. Für die Heilbehandlung der Dictyokaulose können gegen adulte und juvenile Stadien wirksame Medikamente eingesetzt werden (z.B. Levamisol®, (Pro) Benzimidazole, Netomin®, Ivermectin®). Diese Präparate sind hochwirksam und können somit durch akute Lungenwurmerkrankung bedingte Ausfälle meist verhindern (H. Mehlhorn, D. Düwel, W. Raether (1993) Diagnose und Therapie der Parasitosen von Haus-, Nutz- und Heimtieren. 2. Aufl. Gustav Fischer Verlag, S. 223-227). Bei einer prophylaktischen/metaphylaktischen Behandlung lassen die Wirkstoffe aufgrund ihrer radikalen Wirksamkeit möglicherweise die Auseinandersetzung des Parasiten mit dem Immunsystem des Wirtes und somit die Ausbildung und Aufrechterhaltung einer belastbaren (Teil) Immunität nicht zu. Die Tiere sind dann einer Infektion im zweiten Weidejahr ungeschützt ausgesetzt (COBS, D.E., S.R. Pitt, J. Förster, M.T. Fox (1987) Res. Vet. Sci. 43:273-275).

Deshalb wird in letzter Zeit aus epidemiologischer Sicht immer häufiger eine Immunisierung der Kälber der ersten Weidesaison gefordert, entweder durch geringgradige subklinische Infektion oder durch Vakzinierung. Zur Zeit steht nur eine Lebendvakzine in Form röntgenattenuierter Larven zur Verfügung, die Grundimmunität hervorruft, die durch weitere natürliche Infektion aufrecht erhalten werden muß (Mehlhorn H., et al., (1993)). Bei ungenügender Nachimmunisierung über natürliche Infektion kommen gelegentlich bei plötzlicher, starker Exposition Durchbrüche mit Husten und Erkrankungen vor. Da die Vakzine selbst im Kühlschrank nur etwa 3 Wochen haltbar ist, muß sie sorgfältig aufbewahrt und rasch eingesetzt werden. Dieses Procedere verbietet einen "flächendeckenden" Einsatz; die Vakzine bleibt deshalb in erster Linie speziellen Endemiegebieten vorbehalten. Aufgrund der mangelnden Stabilität und Qualität ist die Entwicklung definierter Vakzinen (Subunitvakzine) erforderlich. Es stellte sich nun die Aufgabe, die aufgeführten Nachteile der derzeigen Vakzinierungsmethode durch Bereitstellung eines neuen, vorteilhaften Impfstoffes zu beseitigen. Diese Aufgabe wurde durch die vorliegende Erfindung gelöst.

Die Erfindung betrifft ein neues, immunogenes, natives Protein, genannt DV 18, welches aus adulten Würmern von Dictyocaulus viviparus isoliert wurde. Seine Immunogenität begründet sich vor allem dadurch, daß es nach subcutaner Applikation im Rind eine Antikörperantwort induziert, die dem Tier Immunschutz verleiht. Ferner kann dieses Protein im ELISA zur retrospektiven Immundiagnose der Dictyokaulose im Rind verwendet werden. DV 18 ist durch folgende physikalische Eigenschaften charakterisiert. Das Protein ist beständig in allen verwendeten Puffern. Eine Verminderung der Immunreaktivität nach Tiefgefrierung (-85°C) des gereinigten Antigens konnte nicht festgestellt werden. Für Antigen DV 18 wurde unter Verwendung eines FPLC-Systems und einer analytischen Gelfiltrationssäule (Superose 12 HR 10/30) ein Retentionsvolumen von 13.5ml gemessen (Elutionspuffer: phosphatgepufferte Kochsalzlösung pH 7.4). Das Protein besitzt keine N-glykosidisch gebundenen Kohlenhydratstrukturen vom "high mannose"-oder "Hybrid"-Typ, keine O-glykosidisch gebundenen Kohlenhydratketten und keine komplexen, sialysierten Kohlenhydratketten und ist offensichtlich nicht glykosyliert. DV 18 hat im SDS-Polyacrylamidgel (Phastgel 8-25%) ein geschätztes Molekulargewicht von ca. 17 500 dalton. Der isoelektrische Punkt von DV 18 liegt im Bereich von 5.2-5.85.

Eine Sequenzierung enzymatischer Peptide von DV 18 ergab folgende Teilsequenzen:
- TPPAGVFYQGXSATPIA: (SEQ ID NO.: 1)
N-terminal schließt sich an diese Sequenz die Aminosäure D, N, S, G oder R an.
- XLGVDPASGVLDPKEATLMA: (SEQ ID NO.: 2)
- NLPIEYNP: (SEQ ID NO.: 3)
- AFRREWFQGDG(M)VRRK: (SEQ ID NO.: 4)
- DVFNAPYDDK: (SEQ ID NO.: 5)
- XPPAGVFYQGWSATPIANGSLG: (SEQ ID NO.: 6)
N-terminal schließt sich an diese Sequenz die Aminosäure D S, G oder A an.
- TYHIK: (SEQ ID NO.: 7)
N-terminal schließt sich an diese Sequenz die Aminosäure D oder H an.
- IINASGRRIGWAIK: (SEQ ID NO.: 8)

Als biologische Eigenschaft ist die Entwicklungshemmung von Dictyocaulus viviparus im Rind nach Vakzination herausragend.

Die Erfindung hat daher zum Gegenstand ein immunogenes Protein mit protektiver Wirkung welches aus adulten Würmern des Lungenwurms Dictyocaulus viviparus isoliert wird und welches bevorzugt ein Molekulargewicht von 16000 - 19000 Da, einen isoelektrischen Punkt zwischen 5.2 und 5.8, keine Glykosylierung und eine N-terminale Aminosäureteilsequenz gemäß Tabelle 1 aufweist.

Ein vorzugsweiser Gegenstand der Erfindung ist ein Protein, welches ein Molekulargewicht von 17 500 ± 1 500 Da und/oder einen isoelektrischen Punkt von 5.5 hat.

Ein weiterer Gegenstand der Erfindung ist ein Verfahren zur Isolierung eines Proteins bei dem die Isolierung mit Hilfe von dem Fachmann bekannten Extraktionsmethoden und chromatographischen Methoden durchgeführt wird.

Ein weiterer Gegenstand der Erfindung ist eine DNA, kodierend für ein Protein wie oben beschrieben. Dabei ist insbesondere die Degeneriertheit des genetischen Codes in Betracht zu ziehen.

Ferner ist Gegenstand der Erfindung ein Verfahren zur Isolierung der genannten DNA, bei welchem
a) degenerierte Oligonukleotide, enthaltend eine DNA-Sequenz, kodierend für Teile des erfindungsgemäßen Proteins,
b) die gemäß a) hergestellten Oligonukleotide radioaktiv oder nicht-radioaktiv markiert werden und
c) aus einer cDNA-Bank, hergestellt aus Dictyocaulus viviparus, cDNA-Klone isoliert werden, die mit den nach b) hergestellten Hybridisierungsproben unter stringente Bedingungen hybridisieren.

Ein weiterer Gegenstand der Erfindung ist ein Verfahren zur Isolierung der genannten DNA, bei welchem
a) PCR-Primer, enthaltend eine DNA-Sequenz, kodierend für Teile des erfindungsgemäßen Proteins, oder enthaltend eine Oligo-dT-Sequenz hergestellt werden,
b) mit den so erzeugten PCR-Primeren aus einer cDNA-Bank, hergestellt aus Dictyocaulus viviparus, PCR-Fragmente generiert werden,
c) welche kloniert und nach gängigen Methoden analysiert werden und
d) zur Vervollständigung der cDNA-Sequenz durch Hybridisierungsverfahren wie oben beschrieben an Stelle der degenerierten Oligonukleotide verwendet werden.

Das zuletzt beschriebene Verfahren kann auch so abgewandelt werden, daß für die PCR-Reaktion als Matrize RNA benutzt wird, die in einem zusätzlichen Schritt zunächst revers transkribiert und der so entstandene cDNA-Erststrang zur PCR verwendet wird.

Ein weiterer Gegenstand der Erfindung ist ein rekombinantes Protein, enthaltend die Aminosäuresequenzen gemäß Tabelle 1, welches vorzugsweise durch Expression einer wie oben beschrieben erhaltenen cDNA in Prokaryonten oder Eukaryonten und Aufreinigung nach dem Fachmann bekannten Methoden erhältlich ist.

Ebenfalls Gegenstand der Erfindung ist ein immunchemisches Verfahren unter Verwendung des oben beschriebenen Proteins von D. viviparus, mit welchem die Menge an DV18 spezifischen Antikörpern im Blut von Rindern bestimmt wird, indem man mit DV18 beschichtete ELISA-Platten mit zu untersuchendem Rinderserum inkubiert und gegebenenfalls gebildete DV18/Antikörperkomplexe durch Peroxidase-konjugierte, polyklonale Antikörper und eine entsprechende, dem Fachmann bekannte Farbreaktion nachweist.

Ein anderer Gegenstand der Erfindung ist die Verwendung des oben beschriebenen Proteins von D. viviparus als Vakzine, verbunden mit einem Carrier oder Adjuvans und ggf. Hilfsstoffen zur Immunisierung von Rindern gegen Dictyokaulose.

Ein anderer Gegenstand der Erfindung ist ein Diagnostik-Kit, enthaltend das oben beschriebene Protein von D. viviparus.

Schließlich ist ein Gegenstand der Erfindung eine Vakzine, enthaltend das oben beschriebene Protein von D. viviparus sowie einen Carrier, ein Adjuvans sowie ggf. Hilfsstoffe.

Die Erfindung wird nun anhand der Figur und der Beispiele näher erläutert, ohne darauf beschränkt zu sein. Die Tabelle und die Figur sind wie folgt beschrieben:
- Tabelle 1:: Teil-Aminosäuresequenzen des isolierten DV18-Proteins aus Dictyocaulus viviparus.

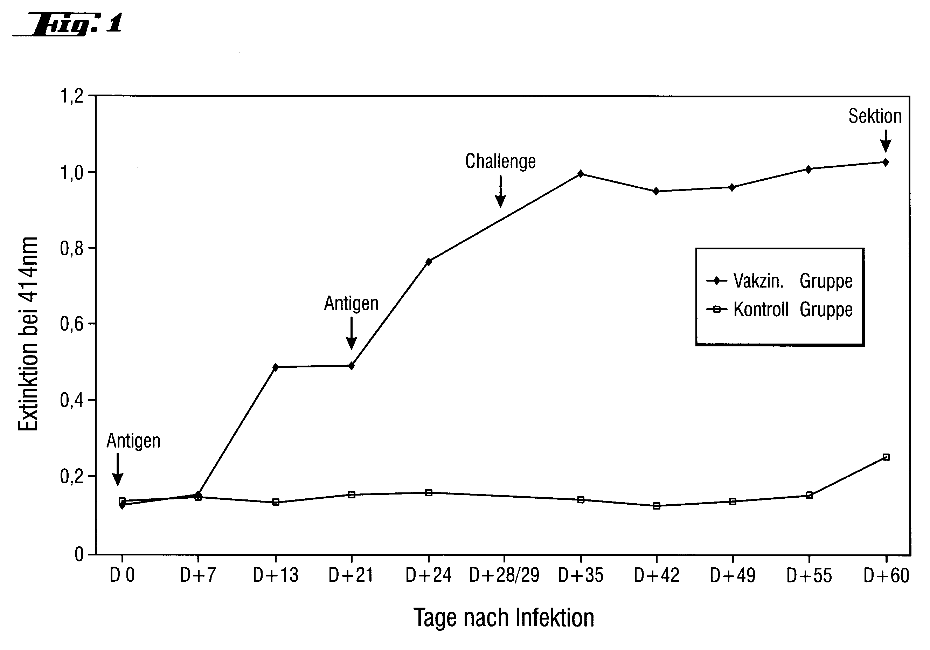

- Figur 1:: Nachweis protektiver, Dictyocaulus spezifischer Antikörpertiter ohne und mit vorheriger Vakzination mit Antigen DV18. Y-Achse: Wellenlänge: 414 nm; X-Achse: Tage. Die Antigengaben erfolgten zu Beginn und am 21. Tag, am 28./29. Tag wurden L3-Larven von Dictyocaulus viviparus appliziert (Challenge). Die Antiserumverdünnung zur Messung betrug 1:200. Details zu diesem Versuch sind in Beispiel 11 zu finden.

Für die Identifizierung von Protein DV 18 wurden Normalseren und Infektionsseren von Rindern verwendet, die mit gastrointestinalen Nematoden wie Ostertagia ostertagi und Cooperia oncophora sowie dem Lungenwurm Dictyocaulus viviparus infiziert waren.

Chromatographisch aufgetrennte Proteinfraktionen gewonnen aus homogenisierten, adulten Lungenwürmern wurden mit Hilfe der SDS-Polyacrylamidgelelektrophorese weiter aufgetrennt und auf Immobilon P-Membranen immobilisiert (Semidry-Blotting). Das lungenwurmspezifische Protein DV 18 wurde anschließend mit den spezifischen Infektionsseren detektiert und mittels analytischer Gelfiltrationssäule weiter aufgereinigt. Die Reinheit der Proteinfraktion wurde in silbergefärbten SDS-Polyacrylamidgelen (Phastgele) überprüft. Mittels BCA-Proteinassay wurde die Proteinkonzentration in elektrophoretisch reinen DV 18-Fraktionen bestimmt und bei -85°C tiefgefroren. Helminthennaive Rinder wurden jeweils mit einer definierten Menge von gereinigtem DV 18 vakziniert. Die Rinder wurden 1 Woche nach der zweiten Vakzination mit L3-Larven von Dictyocaulus viviparus belastet (Challenge). Nicht vakzinierte Tiere dienten als Kontrolle. 4 Wochen nach dem Challenge wurden die Rinder geschlachtet, in der Lunge die Anzahl adulter Würmer bestimmt und die Länge der männlichen und weiblichen Würmer gemessen. Als Maß für den Immunschutz wurde die Reduktion der Anzahl adulter Würmer im Vergleich zur nicht vakzinierten Kontrolle definiert.

Gereinigtes DV 18 ermöglicht im ELISA den Nachweis einer Dictyokaulose bis zu 3 Monaten nach Infektion.

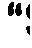 Stringente Bedingungen 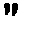 in Zusammenhang mit DNA-Hybridisierung bedeutet in der vorliegenden Anmeldung 6xSSC, 68°C.

Die PCR-Bedingungen sind nach jedem Fachmann bekannten Methoden in Vorversuchen zu bestimmen.

### Beispiel 1: Herstellung von Infektionsseren

Helminthennaive Rinder im Alter von 6 Monaten wurden mit unterschiedlichen Dosen von dritten Larven verschiedener Nematodenspezies (Dictyocaulus viviparus, Ostertagia ostertagi, Cooperia oncophora) infiziert. Die Infektionsdosen betrugen in der Dictyocaulus-Gruppe 2500, 1250 und 500 Larven/Rind; je Dosis wurden 3 Tiere verwendet. Für die Ostertagia-Gruppe wurden Infektionsdosen von 70 000, 30 000 und 15 000 Larven/Rind gewählt. Neben einer nicht infizierten Gruppe (= Negativkontrolle) wurde zusätzlich eine gemischte Gruppe mitgeführt, in der jedes Rind mit 2 500 Dictyocaulus Larven, 10 000 Ostertagia Larven und 10 000 Cooperia Larven infiziert wurde. An den Tagen D0 (= Tag der Infektion), D + 21, D + 40, D + 56, D + 70, D + 84, D + 98 und D + 112 wurden von jedem Rind Serumproben gewonnen, die aliquotiert bei -25°C aufbewahrt wurden. Die Seren wurden zur Identifizierung des Dictyocaulusantigens DV 18 in elektrophoretisch, chromatographisch aufgetrennten Proteinfraktionen sowie zur Beurteilung der Spezifität verwendet.

### Beispiel 2: Gewinnung adulter Lungenwürmer

6 Monate alte, helminthennaive Rinder wurden mit jeweils 5000 dritten Larven von Dictyocaulus viviparus oral infiziert; am darauffolgenden Tag erhielten die Tiere die gleiche Infektionsdosis. 28 Tage nach der Infektion wurden die Rinder geschlachtet und nach der Sektion adulte Würmer aus den Lungen gesammelt. Die Würmer wurden anschließénd 3x mit phosphatgepufferter Kochsalzlösung gewaschen, gewogen und bei -85°C bis zur Aufarbeitung gelagert.

### Beispiel 3: Extraktion von DV 18 aus adulten Lungenwürmern

9.5g gefrorene Wurmmasse wurde unter flüssigem N₂ im Mörser zermahlen und anschließend mit 19ml 0.025M Tris-HCl-Lösung pH 7.4 + 2mM Pefabloc® mittels Ultraturrax homogenisiert. Zur Entfernung grober Gewebebestandteile wurde das Homogenat bei 3010g und 4°C für 15min zentrifugiert und das Pellet verworfen. Der Überstand wurde bei 4°C für 20min bei 39 800g zentrifugiert und der gewonnene Überstand nochmals bei gleicher g-Zahl für 10min zentrifugiert. Der klare Überstand wurde nach Filtration (Porengröße 1.2µm) in 4ml-Aliquote bei -25°C tiefgefroren.

### Beispiel 4: Präparative Gelfiltration

Die in Beispiel 3 erwähnten 4ml-Aliquote wurden einzeln im Pharmacia-FPLC-System mittels präparativer Gelfiltrationssäule (Säulentyp: XK 26/100; Trennmedium: Sephacryl® HR 200, Säulenvolumen: 480ml) aufgetrennt. 0.1M Tris-HCl-Lösung pH 7.4 wurde als Laufmittel verwendet. Die Fraktionen mit den Retentionsvolumina 286-306ml wurden gesammelt und mit Ultrafiltrationsmodulen (centriprep® 10 Fa. Amicon) aufkonzentriert. Mit einem amplifizierten Western Blot wurde Protein DV 18 nachgewiesen.

### Beispiel 5: Western Blot-Analyse

Die aufkonzentrierte Sephacryl HR 200-Fraktion wurde im Verhältnis 1:2 mit reduzierenem SDS-Puffer gemischt; davon wurden jeweils 40µl/Probenvertiefung auf ein SDS-Excelgel (Fa. Pharmacia) aufgetragen. Die Elektrophorese wurde in der Multiphor II-Kammer (Fa. Pharmacia) unter standardisierten Laufbedingungen (600V, 50mA, 30W, Laufzeit: 90min) durchgeführt. Die elektrophoretisch aufgetrennten Proteine wurden mittels "Semidry-Blotting" (Tovey ER, Baldo BA. Electrophoresis. 8, 1987, 384-387) auf Immobilon P-Membranen transferiert (Transferbedingungen: 45min, konstante Stromstärke 0.8mA/cm²) und nach einer 24 stündigen Blockphase mit 3%igem Rinderserumalbumin in Tris gepufferter Kochsalzlösung (TBS) mit einem Dictyocaulus-spezifischem Immunserum (gewonnen am D + 40, siehe Beispiel 1) in einer Verdünnung von 1:20 1 Stunde inkubiert. Als Negativkontrolle wurde Rindernormalserum (Verdünnung 1:20) verwendet. Nach 3 maligem Waschen mit TBS + 0.05% Tween 20 wurde die Blotfolie mit einem Biotin-markierten Ziege anti Rind IgG(H + L) Antikörper (1:500; Fa. Pierce) für 1 Stunde inkubiert. Nach 3-maligem Waschen (TBS + 0.05% Tween 20) erfolgte eine 1 stündige Inkubation mit dem Enzymkonjugat Biotin-Streptavidin-Alkalische Phosphatase (1:2500; Fa. Pierce). Die Substratentwicklung wurde mit dem Substratkit der Fa. Biorad durchgeführt.

### Beispiel 6: Analytische Gelfiltration

Nach dem immunologischen Nachweis von DV 18 wurde das Protein im FPLC-System weiter aufgereinigt. Zu diesem Zweck wurde eine Superose® 12 HR 10/30-Säule der Fa. Pharmacia verwendet; als Laufmittel diente PBS. 300µl der in Beispiel 4 aufkonzentrierten Fraktion wurden pro Lauf auf die Säule aufgetragen; die Flußrate betrug 0.3ml/min. Für DV 18 wurde ein Retentionsvolumen von 13.5ml gemessen.

### Beispiel 7: Reinheitsnachweis und Molekulargewichtsbestimmung

Die FPLC-Fraktion mit einem Retentionsvolumen von 13.5ml wurde mit einem SDS-Polyacrylamidgel (Phast SDS-Gel 8-25%) im Phastsystem unter standardisierten Bedingungen (Fa. Pharmacia) analysiert. Als Molekulargewichtsmarker wurde der "Silver Stain SDS-PAGE Standards, low range"-Kit der Fa. Biorad verwendet. DV 18 wurde nach der Elektrophorese mittels Silberfärbung sichtbar gemacht (Silverstain Kit, Fa. Pharmacia). Die Molekulargewichtsbestimmung wurde mit einem Videodensitometer (Molecular analyst, Fa. Biorad) mittels Auswertungsprogramm "Profile analyst II" durchgeführt. Für DV 18 wurde ein Molekulargewicht von 17 500 ± 1 500 dalton berechnet.

### Beispiel 8: Bestimmung des isoelektrischen Punktes

Gemäß Beispiel 6 isoliertes DV 18 wurde mit Reinstwasser verdünnt und auf vorgefertigte Fokussiergele (IEF Phastgele pH 3-10, Fa. Pharmacia) aufgetragen. Die Fokussierung im Phastsystem erfolgte unter standardisierten Bedingungen. Zwecks Bestimmung des isoelektrischen Punktes von DV 18 wurden Markerproteine mit definiertem, isoelektrischem Punkt ("Biorad pI Kit", pH 3.5-9.3; Fa. Pharmacia) mitgeführt. Es ergab sich ein isoelektrischer Punkt von 5.5 ± 0.3.

### Beispiel 9: Nachweis einer Glykosylierung

Die spezifische Bindung von Lektinen mit bekannter Kohlenhydratspezifität wurde zur Identifizierung von N-Glykanketten oder O-glykosidisch gebundenen Kohlenhydratstrukturen ausgenutzt. Der Digoxigenin-Glykandifferenzierungskit der Fa. Boehringer Mannheim wurde zur Analyse verwendet. Auf Blotfolie (Immobilon P®-Membran) immobilisiertes DV 18 und charakteristische Glykoproteine wurden mittels reversibler Proteinfärbung (Ponceau S-Färbung) sichtbar gemacht, mit Bleistift markiert und anschließend wieder entfärbt. Nach Inkubation mit Digoxigenin-markierten Lektinen wurden Kohlenhydratstrukturen mit einem Alkalische Phosphatase markierten Anti-Digoxigenin-Antikörper nachgewiesen. DV 18 reagierte nicht mit spezifischen Lektinen und ist offensichtlich nicht glykosyliert.

### Beispiel 10: Amminosäuresequenzanalyse

Gemäß Beispiel 6 isoliertes DV 18 (35µg) wurde nach Standardverfahren von einem kommerziellen Anbieter teilsequenziert. Folgende Teilaminosäuresequenzen wurden identifiziert:
- TPPAGVFYQGXSATPIA: (SEQ ID NO.: 1)
N-terminal schließt sich an diese Sequenz die Aminosäure D, N, S, G oder R an.
- XLGVDPASGVLDPKEATLMA: (SEQ ID NO.: 2)
- NLPIEYNP: (SEQ ID NO.: 3)
- AFRREWFQGDG(M)VRRK: (SEQ ID NO.: 4)
- DVFNAPYDDK: (SEQ ID NO.: 5)
- XPPAGVFYQGWSATPIANGSLG: (SEQ ID NO.: 6)
N-terminal schließt sich an diese Sequenz die Aminosäure D S, G oder A an.
- TYHIK: (SEQ ID NO.: 7)
N-terminal schließt sich an diese Sequenz die Aminosäure D oder H an.
- IINASGRRIGWAIK: (SEQ ID NO.: 8)

### Beispiel 11: Nachweis eines protektiven Effekts von DV 18

4 Monate alte, helminthennaive Bullenkälber (Stallaufzucht, koproskopischer Nachweis negativ) wurden mit jeweils 35µg gereinigtem nativem DV 18 subcutan vakziniert. 3 Wochen später erfolgte eine Boostervakzination mit jeweils 20µg Antigen/Rind. Die Tiere wurden 1 Woche nach der 2. Vakzination mit jeweils 2 x 750 L3-Larven von Dictyocaulus viviparus belastet (= Challenge). Nicht vakzinierte Tiere dienten als Kontrolle. 28 Tage nach dem Challenge wurden die Tiere geschlachtet und in der Lunge die Anzahl adulter Würmer bestimmt; gleichzeitig wurde die Länge intakter männlicher und weiblicher Würmer gemessen. Mit Beginn des Vakzinationsversuchs erfolgte eine wöchentliche Blutentnahme und Serumgewinnung. Die spezifischen Antikörpertiter wurden im ELISA (siehe Beispiel 12) ermittelt (siehe Figur 1). In der vakzinierten Gruppe (n = 6 Tiere) wurde eine um 80% reduzierte Anzahl adulter Würmer gefunden. Morphologisch waren adulte Würmer im Vergleich zur Kontrollgruppe signifikant kleiner (Reduktion 40%).

### Beispiel 12: ELISA zum serologischen Nachweis einer Dictyokaulose

ELISA-Platten ("Maxisorb®" der Fa. Nunc) wurden mit einer Konzentration von 5µg DV 18/ml PBS beschichtet; das Reaktionsvolumen betrug 100µl pro Vertiefung. Nach einer Inkubation von 1 Stunde bei 37°C wurden die Platten 3 mal im ELISAwasher® (Fa. Biorad) gewaschen, das Waschvolumen pro Vertiefung betrug 200µl. Als Waschlösung wurde Reinstwasser + 0.1% Tween 20 verwendet. Unspezifische Bindungsstellen wurden durch Inkubation (3 Stunden bei Raumtemperatur) mit einem proteolytischen Gemisch von Gelatine (Fa. Boehringer Mannheim) blockiert. Die Inkubation erfolgte auf einem Mikrotiterplatten-Schüttler bei einer Schüttelfrequenz von 300rpm. Nach 3-maligem Waschen wurden die Vertiefungen mit einer 1:200-Verdünnung der Serumproben beschickt; die Seren wurden unmittelbar vor Gebrauch aufgetaut, gut durchmischt und anschließend mit sterilem PBS verdünnt. Die Serumproben wurden unter Schütteln 1 Stunde bei Raumtemperatur inkubiert. Nach 3-maligem Waschen wurden Peroxidase-konjugierte, polyklonale Kaninchenantikörper gegen bovines IgG (Fc-Fragment spezifisch; Fa. Dianova) in einer Verdünnung von 1:10 000 verwendet, um Dictyocaulus-spezifische Antikörper nachzuweisen; die Inkubation dauerte 30min. Die Platten wurden anschließend 4 mal gewaschen und dann mit Substratlösung inkubiert (Zusammensetzung: 5ml 10-fach konzentrierter ABTS-Puffer (Fa. Boehringer Mannheim) + 45ml Reinstwasser + 1 Tablette ABTS). Die Substratentwicklung erfolgte bei Raumtemperatur und wurde im ELISA-Reader alle 10min bei 414nm überwacht. Dictyocaulus-spezifische Antikörper waren im ELISA frühestens 28 Tage nach einer Lungenwurminfektion nachweisbar und konnten 3 Monate nach der Infektion noch sicher nachgewiesen werden. Kreuzreaktionen mit gastrointestinalen Nematoden wie Ostertagia ostertagi und Cooperia oncophora konnten nicht festgestellt werden.

**Tabelle 1**

| | |
|---|---|
| TPPAGVFYQGXSATPIA | (SEQ ID NO.: 1) |
| N-terminal schließt sich an diese Sequenz die Aminosäure D, N, S, G oder R an. | |
| XLGVDPASGVLDPKEATLMA | (SEQ ID NO.: 2) |
| NLPIEYNP | (SEQ ID NO.: 3) |
| AFRREWFQGDG(M)VRRK | (SEQ ID NO.: 4) |
| DVFNAPYDDK | (SEQ ID NO.: 5) |
| XPPAGVFYQGWSATPIANGSLG | (SEQ ID NO.: 6) |
| N-terminal schließt sich an diese Sequenz die Aminosäure D S, G oder A an. | |
| TYHIK | (SEQ ID NO.: 7) |
| N-terminal schließt sich an diese Sequenz die Aminosäure D oder H an. | |
| IINASGRRIGWAIK | (SEQ ID NO.: 8) |

## Patentansprüche

1. Immunogenes Protein (DV18) mit protektiver Wirkung, dadurch gekennzeichnet, daß das Protein aus adulten Würmern des Lungenwurms Dictyocaulus viviparus isoliert wird.

2. Protein, gemäß Anspruch 1, dadurch gekennzeichnet, daß das genannte Protein ein Molekulargewicht von 16 000 - 19 000 dalton, einen isoelektrischen Punkt zwischen 5.2 und 5.8, keine Glykosylierung und Aminosäuresequenzen gemäß Tabelle 1 aufweist.

3. Protein, gemäß einem der Ansprüche 1 bis 2, dadurch gekennzeichnet, daß das genannte Protein ein Molekulargewicht von 17 500 ± 1 500 dalton hat.

4. Protein, gemäß einem der Ansprüche 1 bis 3, dadurch gekennzeichnet, daß das genannte Protein einen isoelektrischen Punkt von 5.5 hat.

5. Verfahren zur Isolierung eines Proteins, gemäß einem der Ansprüche 1 bis 4, dadurch gekennzeichnet, daß die Isolierung mit Hilfe von Extraktionsmethoden und chromatographischen Methoden durchgeführt wird.

6. DNA, kodierend für ein Protein gemäß einem der Ansprüche 1 bis 4.

7. Verfahren zur Isolierung einer DNA gemäß Anspruch 6, dadurch gekennzeichnet, daß
a) degenerierte Oligonukleotide, enthaltend Teile der DNA-Sequenz gemäß Anspruch 6,
b) die hergestellten Oligonukleotide radioaktiv oder nicht-radioaktiv markiert werden und
c) aus einer cDNA-Bank, hergestellt aus Dictyocaulus viviparus, cDNA-Klone isoliert, die mit den nach b) hergestellten Hybridisierungsproben unter stringente Bedingungen hybridisieren.

8. Verfahren zur Isolierung einer DNA gemäß Anspruch 6, dadurch gekennzeichnet, daß
a) PCR-Primer, enthaltend Teile der DNA-Sequenz gemäß Anspruch 6 davon oder enthaltend eine Oligo-dT-Sequenz, hergestellt werden,
b) mit den so erzeugten PCR-Primern aus einer cDNA-Bank, hergestellt aus Dictyocaulus viviparus, PCR-Fragmente generiert werden,
c) welche kloniert und nach gängigen Methoden analysiert werden und
d) zur Vervollständigung der cDNA-Sequenz durch Hybridisierungsverfahren gemäß Anspruch 8 an Stelle der degenerierten Oligonukleotide verwendet werden.

9. Verfahren gemäß Anspruch 8, dadurch gekennzeichnet, daß für die PCR-Reaktion als Matrize RNA benutzt wird, die in einem zusätzlichen Schritt zunächst revers transkribiert und der so entstandene Erststrang zur PCR verwendet wird.

10. Rekombinantes Protein, enthaltend Teil-Aminosäuresequenzen gemäß Tabelle 1.

11. Rekombinantes Protein, erhältlich durch Expression einer nach einem der Ansprüche 7 bis 9 erhaltenen cDNA in Prokaryonten oder Eukaryonten und Aufreinigung.

12. Immunchemisches Verfahren zur Bestimmung der Menge von DV 18 spezifischen Antikörpern im Blut von Rindern unter Verwendung eines Proteins, gemäß einem der Ansprüche 1 bis 4, 10 oder 11, dadurch gekennzeichnet, daß mit DV 18 beschichtete ELISA-Platten mit zu untersuchendem Rinderserum inkubiert werden und gegebenefalls gebildete DV 18/Antikörperkomplexe mit Peroxidase-konjugierten, polyklonalen Antikörpern und einer Farbreaktion nachgewiesen werden.

13. Verwendung eines Proteins, gemäß einem oder mehreren der Ansprüche 1 bis 4, 10 oder 11 als Vakzine verbunden mit einem Carrier oder Adjuvans und ggf. Hilfsstoffen zur Immunisierung von Rindern gegen Dictyokaulose.

14. Diagnostik-Kit enthaltend ein Protein gemäß einem der Ansprüche 1 - 4, 10 oder 11.

15. Vakzine, enthaltend ein Protein gemäß einem der Ansprüche 1 - 4, 10 oder 11 sowie einen Carrier, ein Adjuvans sowie ggf. Hilfsstoffe.
